# EUROPEAN PATENT APPLICATION

(11) **EP 4 846 986 A2**
(43) Date of publication of application: **02.12.2026**
(21) Application number: 26192342.9
(22) Date of filing: 20.03.2019
(51) Int. Cl.: A61F 5/445

(54) **A MEDICAL SYSTEM, A MEDICAL DEVICE AND A BODY WASTE COLLECTING SYSTEM**

(30) Priority: 21.03.2018 DK PA201800132; 30.01.2019 DK PA201900131
(62) Document number(s) of the earlier application(s) in accordance with Art. 76 EPC: 19714542.8 / 3 768 202
(71) Applicant: Furine ApS, 3320 Skævinge (DK)
(72) Inventor: Thomsen Nielsen, Brian, 3320 Skævinge (DK)
(74) Representative: Münzer, Marc Eric

(57) **Abstract**

A medical system (100) comprising: a solution comprising a first silicone adhesive (301) dissolved in a solvent suitable for application to the skin; said first silicone adhesive solution is adapted to leave an adhesive film (310) on the skin after application, a medical device (200) having a surface comprising a second silicone adhesive (210) comprising a cross-linked silicone gel. The second silicone adhesive (210) has a HexaMethyIDi-SilOxane absorption (HMDSO) of 1100 to 2500%.

## Description

### FIELD OF THE INVENTION

The present specification discloses multiple inventions. A first invention relates to a medical system, a method for producing a medical system and a method for attaching a medical device to a skin surface. Furthermore the first invention relates to a dual adhesive system for attaching a medical device to the skin. A second invention relates to a body waste collecting system. The current claim set is directed to the first invention. For the sake of this specification, the term "body waste" should be interpreted as faecal waste or urinary waste. Additional inventions are also disclosed in this specification.

### BACKGROUND OF THE INVENTION

Many Medical Devices are adhered to the skin. Examples of these are body waste collecting devices (for example perianal and peristomal faecal collecting systems and urine collecting systems), blood collecting devices and wound care devices.

Some devices of the above must stay on even if the adhesive bond towards the skin is stressed via load or unintended pull. For example, adhesive Urisheaths are fastened additionally via the use of adhesive strips between the sheath and the penile skin. In Negative Pressure Wound Therapy the attachment of the wound dressing may be supported by cross linked silicone gel strips between the adhesive film and the skin.

Some devices are adhered to sensitive skin. In this case a liquid film can be applied between the skin and the adhesive. The film is in principle a polymer solubilized in a volatile solvent. When the solvent is evaporated, a thin film layer remains coated on the skin. The medical device is then attached on this film. The most commonly used skin-protective film is Cavilon from 3M. Furthermore WO2014096273 describes a non-adherent skin-protective film made of silicone.

When a strong attachment to the skin is needed, these skin-protective films are made adhesive. Examples of such films include Dow Corning BIO-PSA 7-4401; Dow Corning BIO-PSA 7-4501 and Dow Corning BIO-PSA 7-4601.

However in some cases the adhesive force attaching the medical device to the skin remains too low and there exists a need for an effective and cost effective manner to attach a device to the skin; the attachment supporting a reduction in the amount of leakages and increases the comfort for the user.

The present invention is in particular useful on users that have a need of a strong attachment to the body, but also need frequent changes of at least a portion of the device. One example of such users are ostomy patients and patients with liquid faecal output (BSC type 7) which are being given substantial medical treatment (for example strong antibiotics), for example patients in intensive care units.

### SUMMARY OF THE INVENTION

In a first aspect the first invention relates to a medical system (100) comprising:
a. a solution comprising a first silicone adhesive (301) dissolved in a solvent suitable for application to the skin; said first silicone adhesive solution is adapted to leave an adhesive film (310) on the skin after application,
b. a medical device (200) having a surface comprising a second silicone adhesive (210) comprising a cross-linked silicone gel; and
characterized in that the second silicone adhesive (210) has a HexaMethylDiSilOxane absorption (HMDSO) of 1100 to 2500%.

In a second aspect, the first invention relates to a method for producing a medical system (100) as defined herein, the method comprising the steps of:
a. providing a solution comprising a first silicone adhesive (301) dissolved in a solvent suitable for application to the skin; said first silicone adhesive solution is adapted to leave an adhesive film (310) on the skin after application ;
b. providing a medical device (200) having a surface comprising a second silicone adhesive (210) comprising a cross-linked silicone gel having a HexaMethylDiSilOxane absorption (HMDSO) of 1100 to 2500%, and
c. packing at least the medical device (200), and preferably the medical system (100).

In a third aspect, the first invention relates to a method for attaching a medical system (100) as defined herein to a skin surface (900), the method comprising the steps of:
a. applying a solution comprising a first silicone adhesive (301) dissolved in a solvent suitable for application to the skin on the skin surface (900);
b. evaporating a sufficient amount of the solvent to leave an adhesive film (310) on the skin; and
c. applying a second silicone adhesive (210) comprising a cross-linked silicone gel having a HexaMethylDiSilOxane absorption (HMDSO) of 1100 to 2500%, comprising a medical device (200), to at least a portion of the adhesive film (310) on the skin.

In a fourth aspect, the first invention relates to a dual adhesive system for attaching a medical device (200) to the skin (900), said dual adhesive system comprising:
a. a solution comprising a first silicone adhesive (301) being dissolved in a solvent for application on the skin; said first adhesive after application on the skin being adapted to leave an adhesive film (310) on the skin.
b. a second silicone adhesive (210) comprising a cross-linked silicone gel; and adapted to be attached to a medical device (200); and
characterized in that the second silicone adhesive (210) has a HMDSO-absorption of 1100 to 2500%.

In a fifth aspect, the current specification discloses a body waste collecting system as one embodiment of a medical system according to the first aspect. However, the body waste collecting system as disclosed herein, could also be provided as a system using a different type of adhesive. Since the body waste collecting system disclosed in this specification has novel and inventive features independent of the type of adhesive used, the body waste collecting system is described as an independent invention, and is called the second invention in this specification.

The specification also discloses a novel type of applicator suitable for applying an attachment member of a body waste or faecal collecting system to a user. Likewise, the specification also discloses a medical system comprising a body waste or faecal collecting system with an attachment member and an applicator for applying the attachment member of a body waste or faecal collecting system to a user.

In the following, the inventions will be described in greater detail with reference to embodiments shown by the enclosed figures. It should be emphasized that the embodiments shown are used for example purposes only and should not be used to limit the scope of the inventions.

### BRIEF DESCRIPTION OF THE FIGURES

Figure 1 discloses a cross sectional view of an example of a medical system (100) according to the first aspect of the first invention prior to use.
Figure 2 discloses a cross sectional view of an example of a medical system (100) according to the first aspect of the first invention when being applied on the skin of a user.
Figure 3 discloses a cross sectional view of a schematic illustration of a test setup according to Test Method A (800).
Figure 4 discloses a cross sectional view of a schematic illustration of a test setup according to Test Method B (700).
Figure 5 discloses a picture of the leather used in Test Method B (700) and Test Method C.
Figure 6 discloses a graph displaying the results from a test article tested in Test Method C.
Figure 7 schematically shows a general overview of the different components of a faecal collecting system. .
Figure 8 shows an exploded perspective view of one embodiment of an applicator and a part of an attachment member of a faecal collecting system as shown in figure 7.
Figure 9 shows a partial cross sectional view through the longitudinal plane of the applicator and attachment member of figure 8 with an adhesive applied.
Figure 10 shows a cross section view through the transverse plane of the attachment member of figure 8 with adhesive applied.
Figures 11-13 show perspective, left and front views respectively of another embodiment of an applicator for use with a faecal collecting system as shown in figure 7.
Figures 14-16 show perspective, left and front views of the applicator of figures 11-13 with a portion of an attachment member and conduit.
Figure 17 schematically shows a cross section through the applicator and tubular section of the embodiment of figure 14-16.
Figure 18 shows a perspective view of another embodiment of an attachment member.
Figure 19 shows a side cross sectional view of the attachment member of figure 18.
Figure 20 shows a detailed portion of the side cross sectional view of figure 18 according to the circle XX defined in figure 19.
Figure 21 shows a perspective view of another embodiment of an attachment member.

### Detailed description of the inventions

The first aspect of the first invention pertains to a medical system (100) comprising:
a. a solution comprising a first silicone adhesive (301) dissolved in a solvent suitable for application to the skin; said first silicone adhesive solution is adapted to leave an adhesive film (310) on the skin after application,
b. a medical device (200) having a surface comprising a second silicone adhesive (210) comprising a cross-linked silicone gel; and
characterized in that the second silicone adhesive (210) has a HexaMethylDiSilOxane absorption (HMDSO) of 1100 to 2500%.

In the context of the first invention, the term "HMDSO-absorption" (HexaMethylDiSilOxane) is measured according to test method A (Example 1). Cross-linked silicone adhesive gels with high HMDSO-absorption has a lower degree of cross-linking while cross-linked silicone adhesive gels with lower HMDSO-absorption has a higher degree of cross-linking.

It is known to use dual adhesive constructions. WO2014/096273 describes on page 3 line 11-16 such dual adhesive construction and that they may be troublesome. However, if such strong adhesion is required, for instance when attaching a large external breast prosthesis, there is no teaching of how such a system is designed.

In the first invention, it has surprisingly been found that some dual-adhesive system have a synergistic effect while others do not. The present specification discloses, that dual adhesive systems comprising a second silicone adhesive with an optimized cross-linking degree or HMDSO-absorption increase significantly in bonding strength compared to when using the second adhesive alone, while other dual adhesive systems do not. Surprisingly, this synergistic optimum of adhesive bonding strength in dual adhesive systems is not identical to the optimum when using a single adhesive system based on a second adhesive alone.

The second silicone adhesive may be any type of silicone gel that exercises adhesive properties. In one embodiment of the first aspect of the present invention, the second adhesive is a gel of PolyDiCarbonylSiloxane (PDCS, silicone). Examples of such PDCS include PolyDiMethylSiloxane, PolyDiEthylSiloxane, PolyDiPropylSiloxane or mixtures thereof.

In the first invention, the second silicone adhesive contributes to the overall adhesive power and to the flexibility of the adhesive bond between the skin and the medical device. Consequently, altering the properties of the second silicone adhesive impacts the usefulness of the first invention.

Some medical device systems require both an elastic seal to deal with body movements or external force applied to the device but also a sufficiently strong adhesion to the skin to stay in place.

Useful second adhesives according to the first invention have an optimal cross-linking degree. They do not have a too low cross-linking degree, as this results in a low integrity and therefore difficult to remove and easy to tear. They are not too cross-linked, as this result in a low adhesion power and reduced flexibility.

Cross-linking degrees are difficult and almost impossible to measure by analytical means (branching degree, length and structure of polymer backbone and other chemical structures in the gel-matrix blur the analytical picture). HMDSO-absorption is therefore used in the present specification as a measure of crosslinking, as it is easy to measure and an equivalent way of describing the cross-linking degree.

In some embodiments of the first aspect of the first invention, the second adhesive has a HMDSO-absorption of 1300 to 2200 percent. In one embodiment of the first aspect of the first invention, the second adhesive has a HMDSO-absorption of 1500 to 2000 percent. In another embodiment of the first aspect of the first invention, the second adhesive has a HMDSO-absorption of 1600 to 1900 percent.

Typically, gel-type silicone skin adhesives similar to the second silicone adhesive are softened by use of plasticizers such as silicone oil or other plasticizers that are compatible with silicones (PolyDiCarbonylSiloxane (PDCS). Another reason to add silicone oil to the mixture is that it softens the skin and reduces the stress applied to the skin. In fact, plasticizers not only soften the adhesive and the skin but in correct amounts, they also increase the adhesive bond when applied directly on the skin. Consequently, most gel-type silicone skin adhesive contains 25-50 % plasticizers including non-crosslinked polymers.

In the first invention, it has surprisingly been found, that when a medical device is applied on the skin via a dual-adhesive construction, the adhesive bond increases if the second silicone adhesive comprises a reduced amount of plasticizers. A single adhesive system for attaching a medical device to the human skin, will have increased adhesive strength with for example 30% oil or plasticizer excluding unreacted pre-polymers, while a dual adhesive system will experience reduced adhesive strength with similar portion of 30% oil compared to an oil free mixture. For details see test results from Test Method B (test article B0 and B1 compared to test article D0 and D1).

In the present specification, the term "plasticizer" shall be understood as a molecule that is compatible with silicone adhesives but does not participate in the formation of a crosslinked structure via at least one chemical bond. Typically, a plasticizer is a type of inert silicone oil and may be washed out of the adhesive by using a solvent. Also, if a surplus of one reactant (pre-polymer) is present when the gel is formed, the remaining unreacted pre-polymer will act as plasticizers as long as they are not chemically bonded to the network.

As it may be difficult to distinguish the plasticizers from molecules participating in the crosslinked network by analytical methods, one simple method of measuring the amount of molecules participating in the network is to measure the weight percentage of non-extractable matter in the silicone gel (referred to as gel-percentage).

The cross-linking degree of second adhesives according to the first invention, should, as earlier mentioned, be controlled. By keeping the crosslinking degree relatively low, depending on the formulation, unreacted molecules may remain in the matrix and act as plasticizers. Even though these may be undesirable, they may be included in a cost efficient manufacturing perspective. Also bear in mind that they act to soften the adhesive. Therefore, a second adhesive with Gel-percentage 100% may not be the optimum in all situations.

In the first invention, the molecules in the second silicone adhesive that is chemically attached to and participate in the formation of the gel network is measured as the "Gel-percentage".

In the context of the present specification, the term "Gel-percentage" is measured according to Test Method A. Cross-linked silicone adhesive gels with high Gel-percentage have a lower amount of plasticizers, while cross-linked silicone adhesive gels with lower Gel-percentage have a higher amount of plasticizers. For example a gel with 50 % in Gel-percentage, will have a high amount of oil and unreacted prepolymers, while a gel with a Gel-percentage of 80 will probably only contain unreacted pre-polymers (and/or some reacted pre-polymers that do not participate in the molecular gel-network).

In some embodiments of the first aspect of the first invention, the second silicone adhesive (210) has a gel-percentage of 50 to 100. In an embodiment of the first aspect of the first invention, the second adhesive has a gel-percentage of 60 to 100. In another embodiment of the first aspect of the first invention, the second adhesive has a gel-percentage of 65 to 99. In one embodiment of the first aspect of the first invention, the second adhesive has a gel-percentage of 70 to 98.I In one embodiment of the first aspect of the first invention, the second adhesive has a gel-percentage of 75 to 97. In another embodiment of the first aspect of the first invention, the second adhesive has a gel-percentage of 80 to 95.

The degree of crosslinking of the second silicone adhesive impacts its adhesive power and flexibility. If the crosslinking increases, the shore hardness of the second silicone adhesive increases and the tear strength increases, leading to reduced adhesive power and decreased flexibility. Consequently there is a balance between adhesive power and flexibility. On one hand, a strong adhesive power is needed and on the other hand a sufficient flexibility is needed.

The softness and elongation vs. force applied indicate the elasticity and integrity of the adhesive. In the first invention, it may be desirable to use a second silicone adhesive with optimal elasticity and integrity. A too elastic second adhesive will also have lower integrity while a heavily crosslinked adhesive will not have sufficiently elasticity. Such parameters are measured in Test Method C as the Modulus.

In the context of present specification, the term "Modulus" is measured according to Test Method C. Cross-linked silicone adhesive gels with higher Modulus, such as 1.0 N/mm or above as they are not sufficiently elastic, while cross-linked silicone adhesive gels with lower Modulus are not exercising sufficient integrity. In some embodiments of the first aspect of the first invention, the second silicone adhesive has a Modulus of 0.25 to 0.8 N/mm. In some embodiments of the first aspect of the first invention, the second silicone adhesive has a Modulus of 0.3 to 0.7 N/mm. In an embodiment of the first aspect of the first invention, the second silicone adhesive has a Modulus of 0.35 to 0.65 N/mm. In another embodiment of the first aspect of the first invention, the second silicone adhesive has a Modulus of 0.4 to 0.6 N/mm.

In the above, the "modulus" was used to characterize the second adhesive. Another possible way to specify the softness of the second adhesive, either alone or in combination with the "Modulus", is to use the Shore hardness scale. In one embodiment of the first aspect of the first invention, the second adhesive has a shore hardness of less than A30, less than A20, less than A15 or less than A10.

In an embodiment of the first aspect of the first invention the second silicone adhesive has a gel percentage of 75 to 97, a modulus of 0.35 to 0.65 N/mm and a HMDSO adsorption of 1500 to 2000 percent.

In another embodiment of the first aspect of the first invention the second silicone adhesive has a gel percentage of 80 to 95, a modulus of 0.4 to 0.6 N/mm and a HMDSO adsorption of 1600 to 1900 percent.

The required crosslinking density will depend on the molecular weight of silicone backbone, the shorter backbone, the higher crosslinking density to obtain similar adhesive power. Also, some medical devices require a more soft and elastic adhesion while others require strong adhesion.

An embodiment of the first aspect of the first invention relates to a medical system (100) comprising:
a) a first adhesive (301) dissolved in a solvent suitable for application to the skin; said first adhesive is adapted to leave an adhesive film (310) on the skin after application ,
b) a medical device (200) having a surface comprising a second adhesive (210) comprising a cross-linked gel.

Another embodiment of the first aspect of the first invention relates to a medical system (100) comprising:
a) a first adhesive (301) dissolved in a solvent suitable for application to the skin; said first adhesive is adapted to leave an adhesive film (310) on the skin after application ,
b) a medical device (200) having a surface comprising a second adhesive (210) comprising a cross-linked gel, wherein the second silicone adhesive (210) has a gel percentage of 60-100.

Another embodiment of the first aspect of the first invention relates to a medical system (100) comprising:
a) a first adhesive (301) dissolved in a solvent suitable for application to the skin; said first adhesive is adapted to leave an adhesive film (310) on the skin after application ,
b) a medical device (200) having a surface comprising a second adhesive (210) comprising a cross-linked gel, wherein the second silicone adhesive (210) has a Modulus of 0.35 to 0.65 N/mm.

As mentioned, in most embodiments it may be desirable that the adhesive bond is elastic at least to some extent. Such elasticity is provided by the nature and structure of the second adhesive. However, the thickness and amount of second adhesive used also supports and impacts the overall elasticity.

In some embodiments of the first aspect of the first invention, the second silicone adhesive is attached in an average thickness of from 0.5 to 5.0 mm on a surface of the medical device. In another embodiment of the first aspect of the first invention, the second adhesive is attached in an average thickness of from 1.0 to 4.5 mm on a surface of the medical device. In an embodiment of the first aspect of the first invention, the second adhesive is attached in an average thickness of from 1.5 to 4.0 mm on a surface of the medical device (200).

In order to protect the second silicone adhesive during transport, handling and storage, the second silicone adhesive may be protected by a PEEL-off film that is intended to be removed prior to use. Such PEEL-off film may be any non-touch film. In one embodiment of the first aspect of the first invention, the surface area of the second adhesive is covered by a non-touch film. Such non-touch film may be a flouro-silicone coated polyethyelene-film.

The first silicone adhesive is a solubilized adhesive for skin contact and protection, it is preferably provided in a solution of a volatile solvent. The solution is spread onto the skin. Since it is liquid, it fills and fits any skin contour. As the volatile solvent of the solution evaporates, the first adhesive is left in a relatively thin layer on the skin with a large contact area towards the skin. In order to be soluble, the solution comprising the first silicone adhesive is not a gel as chemically crosslinked material do not dissolve but may swell. Instead, the first adhesive comprises a multiplex of separate soluble adhesive molecules (i.e. discrete polymeric molecules).

In some embodiments of the first aspect of the first invention, the first silicone adhesive is intended to leave a layer on the skin (after solvent evaporation), said first adhesive layer having an average thickness of at most 0.5 mm. In another embodiment of the first aspect of the first invention, the first silicone adhesive is intended to leave a layer on the skin (after solvent evaporation), said first silicone adhesive layer having an average thickness of at most 0.3 mm.

Silicone adhesives are in particular low allergenic and skin friendly, why types of these adhesives are preferred as the first adhesive.

In one embodiment of the first aspect of the first invention, the first adhesive is a silicone adhesive. In an embodiment the first adhesive is a silicone adhesive of discrete polymeric silicone molecules.

In one embodiment of the first aspect of the first invention, the first silicone adhesive is selected from the group consisting of discrete molecules of PolyDiCarbonylSiloxane such as PolyDiMethylSiloxane or PolyDiEthylSiloxane.

In some embodiments of the first invention, prior to application on the skin, the first silicone adhesive is solubilized in a solvent. In one embodiment of the first aspect of the present invention, the solvent is HexaMethylDiSiloxane (HMDSO). The boiling point of the solvent is important as the solvent is intended to evaporate on the skin. Or the solvent may be selected from the group consisting of OctaMethylDiSiloxane, DecaMethylSiloxane or other oligomers of MethylSiloxane. Furthermore Ethyl acetate may be used as a solvent.

In one embodiment of the first aspect of the first invention, the solution comprising the first adhesive is positioned in a solvent in a separate compartment.

It may be beneficial for the user to be able to see where the first silicone adhesive solution is applied on the skin.

In one embodiment of the first aspect of the first invention, the first silicone adhesive solution comprises a colourant. In one embodiment of the first aspect of the present invention, the colourant is present in a concentration of 0.8 to 2.0 percent in the solution. The colourant could be present in a concentration of 1.1 to 1.7

The first silicone adhesive solution should comprise a sufficient amount of solvent to be able to fill the skin contour but not too much as the remaining adhesive layer may be too thin. If there is too much solvent, the solution will be low viscous and have a tendency to run and not fill the contours sufficiently.

In one embodiment of the first aspect of the first invention, the concentration of the first silicone adhesive in the solution is from 30 to 75 w/w percent. In another embodiment of the first aspect, the concentration of the first silicone adhesive in the solution is from 40 to 70 w/w percent. In yet another embodiment of the first aspect of the first invention, the concentration of the first adhesive in the solution is from 45 to 65 w/w percent

In another embodiment of the first aspect of the first invention, the concentration of the first silicone adhesive in the solution is from 45 to 65 w/w percent and a colourant is present in a concentration of 0.8 to 2.0 percent in the solution.

In order to ease application of the first silicone adhesive to the skin, it may be desirable to provide a ready to use solution e.g. a stick soaked in the adhesive solution. Such stick could be a swab-stick.

In one embodiment of the first aspect of the first invention, the solution comprising the first adhesive is absorbed in a swab-stick.

When collecting body fluids, it may be desirable to be able to change the body fluid collecting part without removal of the remaining system. In particular it may be desirable to be able to allow the adhesives to stay on the skin as removal may stress or even rupture sensitive skin. Examples of such a system include ostomy bags (where the replaceable part is the collecting bag and the non-replaceable part is the coupling ring comprising the second adhesive) and faecal collecting systems (where the replaceable part is the collecting bag and the non-replaceable part is attached to the perianal skin). Consequently, the collecting unit may be replaced more frequently than the skin attaching part.

In one embodiment of the first aspect of the first invention, the medical device comprises:
a. a non-replaceable part (220); and
b. a replaceable part (225), said replaceable part to be replaced more frequently than the non-replaceable part;
wherein the non-replaceable part comprises the second adhesive.

In one embodiment of the first aspect of the first invention, the non-replaceable part comprises both the first and the second adhesive.

In one embodiment of the first aspect of the first invention, the replaceable part is detachably attached to the non-replaceable part. In one embodiment of the first aspect of the first invention, the replaceable part is detachably attached to the non-replaceable part by adhesive means. In one embodiment of the first aspect of the first invention, the replaceable part is detachably attached to the non-replaceable part by mechanical means.

In one embodiment of the first aspect of the first invention, the replaceable part is a body fluid collecting unit.

Some medical devices donate liquid to the skin or sub-cutaneous or even intravenous. Such liquids may for example be water, blood or medicine.

In one embodiment of the first aspect of the first invention, the replaceable part is a body fluid donating unit.

In one embodiment of the first aspect of the first invention, the system comprises a solvent for supporting the removal the first adhesive when the system or non-replaceable part is removed from the human.

Examples of devices that could be attached to the skin include urinary collecting devices, ostomy devices, faecal collecting devices, blood collecting devices and wound care devices. However, several other devices could also benefit from this adhesive system, including devices that administer liquid to the body or skin.

The second aspect of the first invention relates to a method for producing a medical system (100) as defined herein, the method comprising the steps of:
a. providing a solution comprising a first silicone adhesive (301) dissolved in a solvent suitable for application to the skin; said first silicone adhesive solution is adapted to leave an adhesive film (310) on the skin after application;
b. providing a medical device (200) having a surface comprising a second silicone adhesive (210) comprising a cross-linked silicone gel having a HexaMethylDiSilOxane absorption (HMDSO) of 1100 to 2500%, and
c. packing at least the medical device (200), and preferably the medical system (100).

In one embodiment of the second aspect of the first invention, the method for producing a medical system comprises the steps of:
a. providing a first adhesive (301);
b. providing a second adhesive (210);
c. providing a non-replaceable part (220) of a medical device (200);
d. proving a replaceable part (225) of a medical device (200); and
e. attaching the second adhesive (210) on the non-replaceable part (220) of the medical device (200).

The third aspect of the first invention relates to a method for attaching a medical system (100) as defined herein to a skin surface (900), the method comprising the steps of:
a. applying a solution comprising a first silicone adhesive (301) dissolved in a solvent suitable for application to the skin on the skin surface (900);
b. evaporating a sufficient amount of the solvent to leave an adhesive film (310) on the skin; and
c. applying a second silicone adhesive (210) comprising a cross-linked silicone gel having a HexaMethylDiSilOxane absorption (HMDSO) of 1100 to 2500%, comprising a medical device (200) to at least a portion of the adhesive film (310) on the skin.

The fourth aspect of the first invention relates to a dual adhesive system for attaching a medical device (200) to the skin (900), said dual adhesive system comprising:
a. a solution comprising a first silicone adhesive (301) being dissolved in a solvent for application on the skin; said first adhesive after application on the skin being adapted to leave an adhesive film (310) on the skin.
b. a second silicone adhesive (210) comprising a cross-linked silicone gel; and adapted to be attached to a medical device (200); and characterized in that the second silicone adhesive (210) has a HMDSO-absorption of 1100 to 2500%.

The fifth aspect of the first invention relates to a body waste collecting system as one embodiment of a medical system according to the first aspect of the first invention.

Such a body waste collecting system could comprise the first adhesive in a separate container, while a portion (the medical device) of the body waste collecting system which is designed to direct and collect liquid faecal or urinary matter from a user may comprise the second adhesive on an attachment flange suitable for attachment to the user.

It should be noted that in one embodiment of a body waste collecting system, it can be used together with adhesive systems according to the first invention as described above. However, in other embodiments of a body waste collecting system, other forms of adhesives could be used. .

As such, for the sake of this specification, a body waste collecting system will be described as a second invention, independent from the first invention. However, as mentioned above, the body waste collecting system according to the second invention could be combined with the adhesive systems of the first invention if desired or another form of adhesive. A body waste collecting system in the form of a perianal faecal collecting system similar to the kind described in this specification is also described in more detail in PCT/EP2017/073345 which is incorporated herein by reference in its entirety. Many of the features disclosed in said application are relevant for the system disclosed in this specification.

In one embodiment of a body waste collecting system, an applicator as described below could be included. The applicator as described below discloses advantageous features which are independent of the type of adhesive used and independent of the body waste collecting system used. Hence the applicator described below could form the basis of a divisional application without being limited to the type of adhesive used or to the type of body waste collecting system used.

A body waste collecting system according to the second invention comprises a) an attachment member arranged to be attached to the user, said attachment member comprising an opening which is arranged to be in fluid communication with the anus of the user or with a stoma of the user and an attachment flange which is arranged to encircle said opening and which is arranged to be attached to the perianal or peristomal skin of the user such that a fluid tight seal is established between the attachment flange and the perianal or peristomal skin of the user around the anus or stoma respectively, said attachment flange being provided with a skin friendly adhesive on a skin contacting surface of said attachment flange,, b) a collection bag for collecting the body waste output and c) a tubular section or hollow conduit having a first end and a second end, said first end connected to said attachment member and being in fluid communication with the opening of said attachment member and said second end being in fluid communication with said collection bag.

In one embodiment, the adhesive could be the second adhesive of the first invention. In this case, the first adhesive as described above could be applied to the skin of the user and the second adhesive could be applied to the attachment flange. However any other form of suitable adhesive could also be used. For example, in one embodiment, the first adhesive of the first invention could be applied both to the skin and to the flange of the attachment member.

In another non limiting example, the adhesive on the attachment flange could be applied to the attachment flange in the factory and covered by a protective film for shipping/handling. The adhesive could also be stored in a separate container and applied to the attachment flange by the user or a helper just prior to applying the attachment member to the user. In another option, the adhesive could be applied to the skin of the user just prior to application of the attachment member to the skin of the user. In this case, no adhesive is applied to the attachment member prior to attachment, but once the attachment member is applied to the skin, then a layer of adhesive will be arranged on the skin facing side of the attachment flange. The above described embodiments are not limited to any specific type of adhesive, but could be used with any suitable adhesive, both those described in this specification and with other suitable adhesives provided by the person skilled in the art of adhesives.

It should be noted that the body waste collecting system shown in the drawings is designed to collect faecal waste from the anus of a user. However, the body waste collecting system of the current invention could also be used to collect faecal waste coming from a stoma of a user. Instead of mounting the attachment member to the perianal skin around the anus, the attachment flange could be adhered to the peristomal skin around the stoma of a user. As the stoma typically protrudes from the skin, the stoma can be arranged to fit inside the opening of the attachment member. An ostomy wax or ostomy ring could be applied between the stoma and the attachment member to further seal the connection between the stoma and the attachment member. This type of body waste collecting system could be attached to colostomies, ileostomies and urostomies and collect faecal matter or urinary matter.

In the figures, the attachment flange is shown having a shape where the dorsal and ventral portions are higher than the central portions. This is to ensure that the attachment flange can adhere properly to the Crena Ani and Perineum of the user when collecting faecal matter from the anus. In the case where the attachment member should be designed to be attached to a stoma, the attachment flange could be arranged in a more planar manner. However, in the case where the attachment flange is flexible and stretchable enough, then the same attachment flange could be used to connect to both stoma and anus.

In one embodiment the collecting bag is detachably attached to the outlet of the tubular section, for example via a connecting mechanism.

In order to apply the attachment member of the body waste collecting system to a user, it may be beneficial for the body waste collecting system to comprise an applicator to help apply at least a portion of the attachment member to the user. It may be beneficial that such an applicator somehow supports at least a portion of the attachment flange of the attachment member during application and/or storage.

In one embodiment of the second invention, the body waste collecting system comprises an applicator adapted to support the application of the attachment flange to the perianal or peristomal skin of the user. In one embodiment, the applicator is used to support the application of the attachment flange onto an adhesive film applied to the user in a previous step. In one embodiment, the applicator is used to support the application of the attachment flange directly onto the skin of the user. In one embodiment, the applicator comprises a first portion arranged to support a dorsal portion of the attachment flange and a second portion arranged to support a ventral portion of the attachment flange. In this case, the dorsal and ventral portions respectively can be designed to be pressed into contact with the Perineum and the Crena Ani of a user. In one embodiment, the applicator further comprises a space between the first and second portions such that a tubular section from the attachment member can pass between the first and second portions. In one embodiment, the first and second portions are connected to each other by an annular ring through which the tubular section can pass. In one embodiment, the first and second portions are connected to each other by a side portion arranged on only one side of the applicator allowing the tubular section to pass the side portion. In one embodiment, the first and second portions are connected with a U shaped connector where the bottom portion of the U shaped connector is connected to a handle portion and the upper ends of the U shaped connector are connected to the first and second portions of the applicator.

In one embodiment of the applicator, the applicator is formed with a flattening mechanism which flattens the tubular section of the attachment member . In one embodiment, the flattening mechanism is arranged such that when the tubular section is placed in the flattening mechanism, the cross section of the tubular section taken perpendicularly to the longitudinal axis of the tubular section changes such that a dimension of the cross section which is perpendicular to a line connecting the first and second portions of the applicator decreases. In one embodiment, the first and second portions of the applicator are provided with squeezing elements which are adapted to detachably fixate opposing portions of the conduit of the attachment member to hold it in a flattened manner. In one embodiment, each of the squeezing elements are arranged as two parallel flanges into which a portion of the tubular section can be press fitted. In one embodiment, the distance between the two parallel flanges can be slightly less than twice the wall thickness of the tubular section. In one embodiment, the flattening mechanism could comprise a single squeezing element arranged near the centre of the applicator which squeezes the central portions of the tubular section together, thereby causing the minor axis dimension of the cross section of the tubular section to decrease and the major axis dimension to increase.

Depending on the distance between the squeezing elements and to ease removal of the applicator from the pressed tubular section, it may beneficial to allow the squeezed contact between the applicator and the tubular section to be in a relatively short point. In one embodiment, each of the squeezing elements are arranged as two opposite points. These points may be the most narrow distance between two roughly parallel flanges into which a portion of the tubular section can be press fitted. In one embodiment, the applicator and the tubular section of the faecal collecting system can be designed complementary to each other so that the tubular section can be held more securely in the applicator and also released in a more consistent manner from the applicator when desired. In one embodiment, the tubular section is formed with a ridge on either side of the tubular section near the attachment member running along the longitudinal axis of the tubular section, said ridge engaging with the squeezing elements of the applicator. In one embodiment, the ridges are placed at a distance from the most dorsal and ventral portions of the tubular section.

The ridge and engagement could also be in the perpendicular direction of the longitudinal axis of the tubular section and be a thickening (such as increased wall thickness) on the tubular section. In one embodiment, the tubular section is formed with a thickening near the attachment member running perpendicular to the longitudinal axis of the tubular section, said thickening engaging with the squeezing elements of the applicator.

Since the shape and contour of human beings vary, it is important, that a body waste collecting system is flexible - at least the flange portion should be able to adapt to the shape of the user of the system. This includes that the user may move his or her body and thereby stretch the skin around the anus or stoma. Consequently, the flexibility of the combination of the attachment flange portion and the adhesive can be important.

In the prior art solutions where a faecal collecting system with an attachment flange is attached to the perianal skin with adhesive, the attachment flange is relatively inflexible and relatively unstretchable. It has however now been discovered, that this negatively impacts how well the system remains attached to the user and how comfortable the system is. Hence, in one embodiment of the body waste collecting system, the attachment flange (including the adhesive if applied) has an elongation at break of at least 50%, at least 100%, at least 200% or at least 300%. In one embodiment, the attachment flange (including the adhesive if applied) has an elongation at break between 300% and 1200%.

While a number of specific examples of materials suitable for providing this form of behaviour are provided herein, other forms and types of material should be able to be provided by the person skilled in the art of materials. Hence, the scope of protection should not be limited to the specific compositions of materials mentioned herein.

In one embodiment of the body waste collecting system, the attachment flange is made of an elastomeric material such as TPE. In one embodiment , the attachment flange is made of a silicone material such as Liquid Silicone Rubber. In one embodiment, the attachment flange is a made of a material with a hardness of shore A90 or less, of shore A60 or less, of shore A45 or less or of shore A5 to shore A40. In one embodiment, the attachment flange has an average material thickness of less than 1 mm excluding the adhesive.

Some users have increased skin perspiration (moist evaporation from the surface of the skin), which may be due to any number of different variables or factors. In one example, a user might suffer from fever or a user might have recently damaged some skin. In both these examples, the user will experience increased perspiration. In general, most users will experience some form of sweat or perspiration at certain times. Moisture is known to reduce the adhesive power of systems adhered to the skin.

Some prior art adhesives (such as Hydrocolloid adhesives) deal with skin perspiration via absorption as they to a large extent store condensed moisture in the adhesive matrix itself. As the moisture is absorbed, the adhesive will slowly loose its adhesive power. Other (nonabsorbent adhesive systems) will not be able to absorb any moisture. When the skin perspiration increases, moisture is built-up between the adhesive and the skin, resulting in reduced or lost adhesive power.

When attaching an attachment flange comprising an adhesive to the skin of a user, it may therefore be desirable to provide a system which allows the skin perspire to escape the system. This ensures a more robust connection. For an attachment member of a body waste collecting system, one could allow the attachment flange to have a certain permeability to water vapour. Perspired moisture would then migrate through the flange and into the atmosphere, thereby maintaining the strength of the adhesive joint between the skin and the attachment member. Removal of moisture will also keep the skin underneath the attachment member healthier than if the skin was more occluded. In the product literature, the permeability to water vapour of a product is typically measured as moist vapour transmission rate (MVTR).

In one embodiment of the body water collecting system, the average MVTR of the attachment flange of the attachment member is greater than the normal perspiration of human skin which is typically between 300 and 400 g/m2/24h.

In one embodiment, a first surface area of the attachment flange has an MVTR of at least 500 g/m2/24h, at least 750 g/m2/24h or at least 1000 g/m2/24h. In one embodiment, the first surface area of the attachment flange is at least 5%, at least 10%, at least 25% or at least 50% of the total surface area of the attachment flange.

Relatively high permeability of the attachment flange portion, may for example be achieved by specific choice of materials and/or via the thickness of the materials. In general, the thinner a flange, the higher its permeability.

However, an ultra-thin flange may in some situations be difficult to handle. One way of compensating for both easy handling and high average permeability is to provide a structure with thicker materials in some areas and thinner materials in other areas.

Normally, skin perspiration occurs perpendicular to the skin surface. But it has surprisingly been found, that if a relatively small skin area is occluded, then moist perspiration of the occluded area is transported parallel to the surface of the skin until it reaches a non-occluded skin surface. Such small area may be 2-10 mm wide. So even though an adhesive loses its adhesive force when set on moist skin (or skin becoming moist via perspiration), if the distance to a higher permeability area is low, moisture will be transported to such area and the adhesive power remains high.

In one embodiment, a second surface area of the flange portion has an MVTR of less than 750 g/m2/24h. In one embodiment, a second surface area of the flange portion has an MVTR of less than 500 g/m2/24h or less than 350 g/m2/24h. In one embodiment, the second surface area of the attachment flange is less than 75%, less than 50%, less than 40%, less than 30% or less than 20% of the total surface area of the attachment flange.

In one embodiment, the permeability (and the stretchability and flexibility) of the attachment flange is improved by providing an attachment flange with a pattern of through going holes encircling the opening of the attachment member. In this way a thicker flange material can be used, but the material can be weakened to allow stretching and flexing and to provide permeability by providing the pattern of through going holes passing perpendicularly through the material of the attachment flange. In this case, the attachment member could further comprise a thin film glued to the skin facing side of the attachment flange to cover the holes. A thin layer of adhesive could then be applied to the thin film. In certain cases, a peelable protective film to protect the adhesive could be applied on top of the adhesive to protect it during shipping and handling. The thin film could also be a nonwoven or a woven fabric like material.

In another embodiment, or in combination with the above embodiment, instead of providing through going holes, the attachment flange could be arranged as a flange having a first thickness and then having recesses or areas with a second thickness which is lower than the first thickness arranged on the surface of the flange. In this way, the thicker areas will provide strength and the thinner areas will provide greater flexibility, stretchability and permeability. In this case, the attachment flange does not have any through going openings, and adhesive applied to the skin facing surface of the attachment flange will not progress through the attachment flange. An independent thin film is then not so necessary. In one embodiments, the recesses are located on the surface of the attachment flange facing away from the skin and the skin facing surface is smooth.

Another way of providing the same effect is to provide a thinner flange material and then add ridges onto the flange material. In this way, the ridges will provide strength and the thinner flange material will provide permeability, stretchability and flexability. In one embodiment, the ridges could take the form of ridges extending radially outwardly from the opening combined with ridges which encircle the opening, similar to a spiders web. In another embodiment, the ridges could be in the form of a net structure, for example with horizontal and vertical ridges. In one embodiment, the ridges are located on the surface of the attachment flange facing away from the skin.

In one method of manufacturing an attachment member, the method could start with placing a thin film in the order of 20-100 µm into an injection mould, and then injection moulding the central portion and the tubular portion of the attachment member together with the ridges on the thin film such that the ridges and central portion are overmoulded onto the thin film.

In one embodiment, the adhesive is pattern coated on the attachment flange.

Output from the anus or the stoma may have an undesirable odour. Consequently, if the attachment flange is permeable, such odour may migrate out through the attachment flange. One way of reducing such odour escape is to ensure that the attachment flange has a low permeability in the immediate vicinity of the anal orifice. In one embodiment, the attachment flange comprises a central portion encircling the opening of the attachment member and an outer portion encircling the central portion. In one embodiment, the central portion has an MVTR of less than 750 g/m2/24h.

In one embodiment, the outer portion has an MVTR of at least 750 g/m2/24h and the central portion has an MVTR of less than 750 g/m2/24h. In one embodiment, the outer portion has an MVTR of at least 750 g/m2/24h and the central portion has an MVTR of less than 500 g/m2/24h.

In one embodiment, the average width of the central portion of the attachment flange is from 1 - 12 mm. In one embodiment, the average width of the central portion of the attachment flange is between 2 - 10 mm. In this specification, the width should be understood as the dimension of the attachment flange which is radial to the opening and parallel with the plane of the attachment flange. In one embodiment, the average width of the attachment flange is between 12 and 30 mm, between 14 and 27 mm or between 16 and 24 mm. In another embodiment, the minimum width of the attachment flange is greater than 8 mm, greater than 10 mm or greater than 15 mm.

It should be noted that the embodiments shown in the figures show an attachment flange with a roughly circular outer periphery. However within the scope of the current invention, the outer periphery of the attachment flange could take other forms and does not have to be circular.

It will be appreciated that any combination of features and elements of the above described aspects, inventions and/or embodiments may be combined in any suitable manner.

### Detailed description of the drawings

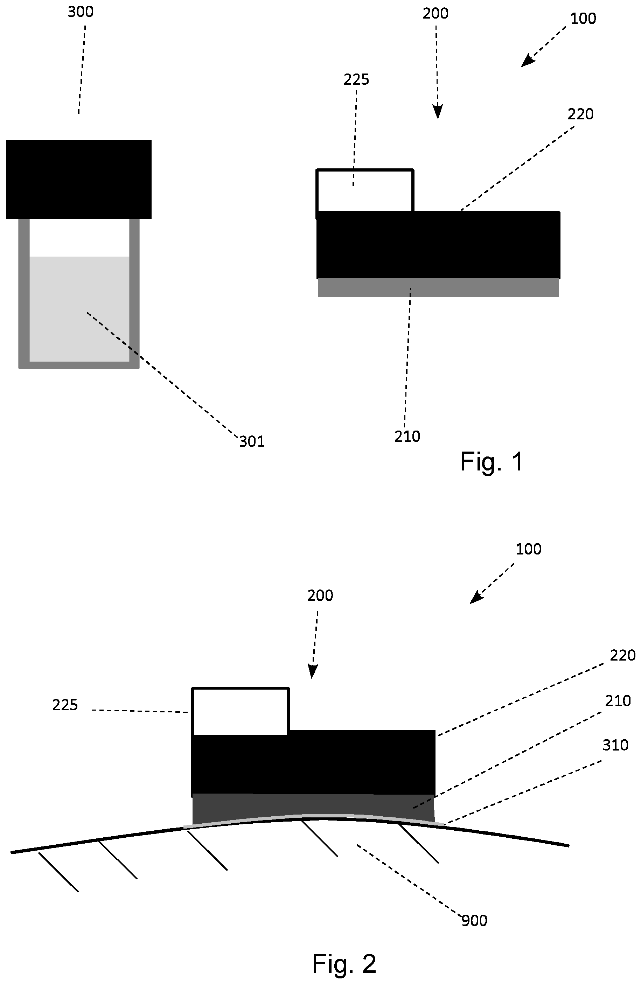

Figure 1 discloses a cross sectional view of an example of a medical system (100) according to the first aspect of the present invention prior to use. In this example, the solution comprising a first silicone adhesive (301) is contained in a small container (300). The user will when applying the first adhesive (301) to the skin, dip an absorbent material for example a swap stick into the container (300) so the first adhesive (301) is absorbed into the swap stick. The user will smear the swap stick on the skin leaving a thin layer of liquid on the skin. The solvent will evaporate and a film of first adhesive (310) will remain on the skin.

The second adhesive (210) is an integral portion of the medical device (200), applied for example on one surface of the device. In this example the medical device (200) comprises a non-replaceable part (220) and a replaceable part (225). In this example, the non-replaceable part (220) comprises the second adhesive (210) and is changed when the second adhesive (210) is removed. The replaceable part in this example (225) is detachably attached to the non-replaceable part (220) via a coupling ring (not disclosed). The intention is to change the replaceable part (225) more frequent than the remaining medical system (100) and device (200).

Figure 2 discloses a cross sectional view of an example of a medical system (100) according to the first aspect of the present invention when being applied on the skin of a user (900). An adhesive film (310) of the first silicone adhesive is in direct contact with the skin (900). The second adhesive (210) comprising the medical device (200) is applied on surface of the adhesive film (310) of the first adhesive (301). The replaceable part (225) of the medical device (200) can be changed without removing the non-replaceable part (220).

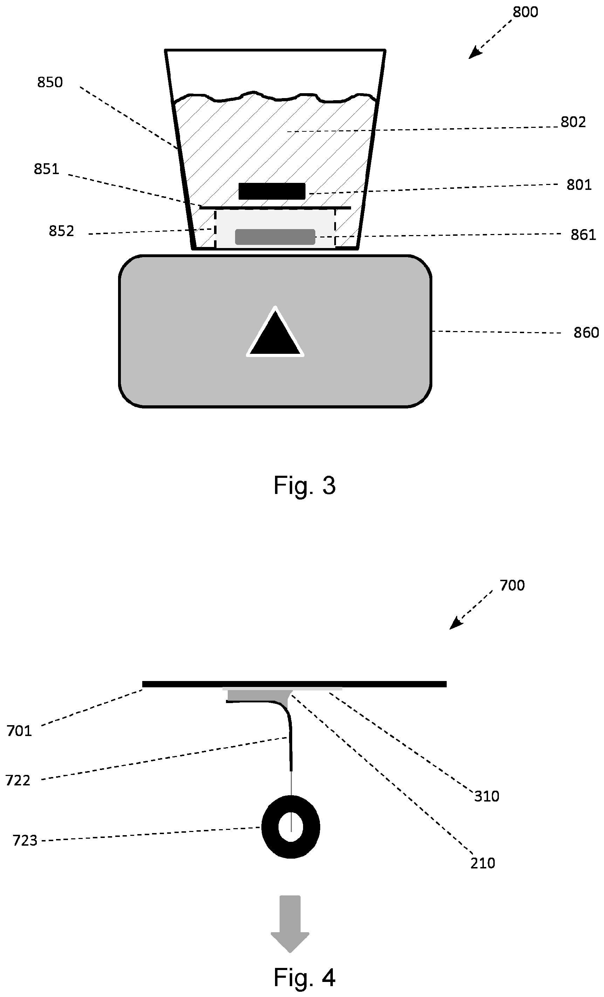

Figure 3 discloses a cross sectional view of a schematic illustration of a test setup according to Test Method A (800). For further details, see description of Test Method A.

Figure 4 discloses a cross sectional view of a schematic illustration of a test setup according to Test Method B (700). In the illustrated test set-up, a dual adhesive system is tested. Test Method B may also be used on a single adhesive system. For further details, see description of Test Method B.

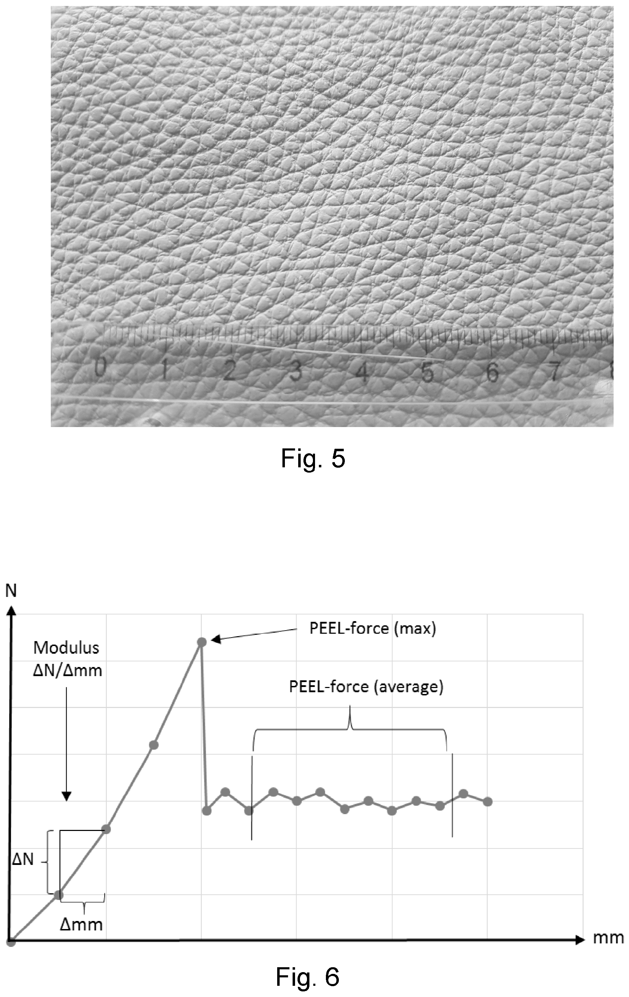

Figure 5 discloses a picture of the leather used in Test Method B (700) and Test Method C. A ruler is included displaying mm and cm.

Figure 6 discloses a graph displaying the results from a test article tested in Test Method C. For further details, see description of Test Method A.

It is to be noted that the figures and the above description have shown the example embodiments in a simple and schematic manner. Many of the specific mechanical details have not been shown since the person skilled in the art should be familiar with these details and they would just unnecessarily complicate this description.

### Examples

### Example A, Second (gel-type) adhesives:

Silicone gels are well known and described. They are produced by many suppliers including Silicone suppliers like companies like Dow Corning, NuSil and Wacker. Examples include Dow Corning MG 7-9900 Soft Skin Adhesive Kit and NuSil MED-6345 (Tacky silicone gel) and SILPURAN^{®} 2114 from Wacker.

One method of making a silicone gel is described in US2007270555 (A1).

If one were to make a Silicone gel of PolyDiMethylSiloxane, a typical process of manufacture include to use pre-polymers of vinyl-functional PolyDiMethylSiloxane (mono, di and/or multivalent) and mix these with pre-polymers of SiH-functional PolyDiMethylSiloxane (mono to multifunctional) and add a platinum catalyst for addition-crosslinking. After reaction an insoluble adhesive Silicone gel will be present. The more multivalent pre-polymers the more crosslinked (and eventually non-tacky) and the more monovalent pre-polymers, the less crosslinked (and more tacky). The needed degree of crosslinking depends on the molecular weight of the pre-polymers and the amount of monofunctional prepolymers (each monofunctional polymer that participate in the network eliminate a potential crosslinking). Furthermore to soften a Silicone gel, Silicone oil may be added.

### Example B, First (soluble) adhesives:

Soluble Silicone adhesive are well known and described. They are produced by many different suppliers including companies like Dow Corning, Examples include Dow Corning MG 2401.

If one were to make a Soluble Silicone adhesive of PolyDiMethylSiloxane, a typical process of manufacture include to use a soluble silicate resin and react this with Silanol endblocked PolyDiMethylSiloxane (via a polycondensation reaction) to form an adhesive condensate.

### Description of the Tests

### Example 1.

### Test Method A (800):

Purpose: To measure GEL-PERCENTAGE, HMDSO-ABSORPTION and wet integrity of cross-linked Silicone adhesive gels.

Description: A cylindrical sample of a Test Article (801) having a diameter of app. 2.5 mm and a thickness of 2-3 mm is prepared. Record the weight of the test article as "start weight". The weight of test article (801) should be between 1.5 and 2.5 grams.

The Test article (801) is placed in a 3-500 ml beaker (850), adding HexaMethylDiSilOxane (HMDSO, 802) corresponding to between 140 to 160 times the weight of the test article. For circulation of the HMDSO a magnet (861) and a magnetic stirrer (860) is used. The magnet (861) is positioned under the Test Article in such a manner that the HMDSO is circulated but the magnet does not physically damage the Test Article via physical contact during stirring. An example of such a Test setup is shown on Figure 3, where the test article (801) is placed on a table (851) with feet (852) allowing the magnet (861), when the magnetic stirrer (860) is turned on, to carefully circulate the HMDSO (802) in the beaker, while the test article remain in place on the table (851) while being immersed into the HMDSO (802).

The beaker is placed under circulation for 48 hours and the test is completed. As the test article (801) may be fragile (depending on the degree of crosslinking), the HMDSO is carefully filtrated away leaving a wet (HMDSO) circular disc. The weight of the wet test article is recorded as "wet weight". The test article is allowed to dry (temperature 20-50 degree celcius) until the weight is stable over 24 hours (this may take 72-144 hours, depending on the temperature). The weight of the dried sample is recorded as "washed weight". The following parameters are calculated:
HMDSO-ABSOPRTION (%) = 100 x ("wet weight" - "start weight") / "start weight" GEL-PERCENTAGE (%) =) 100 x "washed weight" / "start weight"
Wet-integrity score: The cohesiveness of the test article after complet HMDSO-Absorption was evaluated qualitatively based on following:
   Score 0 = Only possible with use of filter paper to determine where the adhesive vs remaining HMDSO was.
   Score 1 = When removing the sample using fingers from the HMDSO-solution the test article received damage.
   Score 2 = When removing the sample using fingers from the HMDSO-solution the test article remained integral.
   Score 3 = When removing the sample using fingers from the HMDSO-solution the test article remained integral. The test article could withstand manual some elongation pull

### Sample description:

| **Test Article** | **Description: Second adhesive** | **Supplier** |
|---|---|---|
| TA-A | Crosslinked Silicone adhesive gel with lower degree of crosslinking (brand name Securin Silicone skin Adhesive BN0104-40). No oil added, but include some unreacted pre-polymers. The gel is made of PolyMethylDiSiloxane according to example A. | Securin ApS |
| TA-B | Crosslinked Silicone adhesive gel similar to TA-A, but with medium degree of crosslinking (brand name Securin Silicone skin Adhesive BN0104-50). No oil added, but includes some unreacted pre-polymers. | Securin ApS |
| TA-C | Crosslinked Silicone adhesive gel similar to TA-A, but with a high degree of crosslinking (brand name Securin Silicone skin Adhesive BN0104-60). No oil added, but includes some unreacted pre-polymers. | Securin ApS |
| TA-D | Crosslinked Silicone adhesive gel identical to TA-B, but added 25% oil. | Securin ApS |
| TA-E | Crosslinked Silicone adhesive gel with very high degree of crosslinking (brand name Mepiseal). Comprising an unspecified amount of Silicone Oil. | Mölnlycke AB |
| TA-F | Crosslinked Silicone adhesive gel with low to medium degree of crosslinking (brand name DOW Corning MG 7-9900 Soft Skin Adhesive). Comprising an unspecified amount of Silicone oil. | Dow Corning |

### Results:

| **Test Article** | **GEL-PERCENTAGE (%)** | **HMDSO-ABSORPTION (%)** | **Wet integrity (score)** |
|---|---|---|---|
| TA-A | 70 | 2000 | 1 |
| TA-B | 85 | 1750 | 2 |
| TA-C | 90 | 1300 | 2 |
| TA-D | 61 | 1650 | 1 |
| TA-E | 80 | 800 | 3 |
| TA-F | 76 | 1800 | 0-1 |

### Test result discussion:

All adhesive gels swells significantly in HMDSO. By comparing TA-A, TA-B and TA-C (similar gels with increasing crosslinking degree), it is seen, that crosslinked Silicone adhesive gels with lower crosslinking degree swells/absorb more HMDSO and become more fragile. Furthermore, the gel-percentage increases with increased crosslinking as the amount of unreacted pre-polymers decrease.

By comparing TA-B, and TA-D it is seen, that by increasing the amount of plasticizer (TA-D has been added 25 % oil), the gel-percentage reduce significantly while the HMDSO-absorption decreases slightly and the wet integrity decreases. A reasoning for this is that the plasticizer act as solvent, why the gels with oil (TA-D) already prior to soaking in HMDSO has been minimally swollen. This also impacts the wet integrity.

### Example 2.

### Test Method B:

Purpose: To measure bonding strength of Silicone adhesives systems to a skin-like surface.

Description: An illustration of the test set-up is shown on figure 4. A 3 mm layer of Cross-linked Silicone adhesive gel (210) is coated onto a fabric cloth with minimal elongation properties (722) is such a manner, that a 25x100 mm test strip (720) comprising an adhesive surface of 25x40 mm is prepared. A piece of buffalo leather (701) of 100x1000 mm - (texture of the leather see figure 5) is cleansed with HMDSO. Depending on the test, a solvent-based adhesive such as a solubilized silicone adhesive according to example B (301) may be applied on the leather (701) in a thickness of app. 0.1-0.5 mm allowing the solvent to evaporate leaving an adhesive film (310) on the leather (701). Depending on the test, the test strip (720) comprising the 25x40 mm surface of Cross-linked Silicone adhesive gel (210) is attached either directly on the leather (701) or on the adhesive film (310) applied on the leather (701), by carefully pushing the test strip (720) onto the leather (as a professional would do on the skin). The leather (701) is fastened horizontally while the leather surface is facing downwards. A 500 grams load is attached to the test strip and released over 1-2 seconds manually. A timer is started simultaneously. The time until the test strip (210) separates from the leather (701) or adhesive film (310) is recorded and time is transferred to a score according to the table below. If the test strip remains on the leather for 15 minutes, the 500 grams load is replaced with a 1000 grams load and the timer is restarted.

**Scoring table**

| **Type** | **Description of Bonding score - Type (categorization)** |
|---|---|
| Type 0 | The Adhesive drops off the leather immediately after release of the 500 grams load (time not measurable, less than 0.5 seconds). |
| Type 1 | The Adhesive drops off the leather after 0.5 but before 5 seconds after release of the 500 grams load. |
| Type 2 | The Adhesive drops off the leather between 5 and 30 seconds after release of the 500 grams load. |
| Type 3 | The Adhesive drops off the leather between 31 and 120 seconds after release of the 500 grams load. |
| Type 4 | The Adhesive drops off the leather between 2 and 6 minutes after release of the 500 grams load. |
| Type 5 | The Adhesive drops off the leather between 6 and 15 minutes after release of the 500 grams load. |
| Type 6 | The Adhesive does not drop off the leather within 15 minutes after release of the 500 grams load. The 500 grams load is replaced with a 1000 grams load. The Adhesive drops off the leather before 5 seconds has passed after release of the 1000 grams load. |
| Type 7 | The Adhesive drops off the leather between 5 and 30 seconds after release of the 1000 grams load. |
| Type 8 | The Adhesive drops off the leather between 31 and 120 seconds after release of the 1000 grams load. |
| Type 9 | The Adhesive drops off the leather between 2 and 6 minutes after release of the 1000 grams load. |
| Type 10 | The Adhesive drops off the leather between 6 and 15 minutes after release of the 1000 grams load. |
| Type 11 | The Adhesive does not drop off the leather within 15 minutes after release of the 1000 grams load. |

### Sample description:

| **Test Article** | **Description of test-set-up** |
|---|---|
| TB-A0 | The gel-adhesive used was the crosslinked Silicone adhesive gel from TA-A. TA-A was applied directly on the leather. |
| TB-A1 | The gel-adhesive used was the crosslinked Silicone adhesive gel from TA-A. Prior to application of TA-A, the leather was coated with Dow Corning MG 2401 from example B (adjusted solid content to 50%), and subsequently, the solvent had evaporated. TA-A was applied on the remaining adhesive film on the leather. |
| TB-B0 | The gel-adhesive used was the crosslinked Silicone adhesive gel from TA-B. TA-B was applied directly on the leather. |
| TB-B1 | The gel-adhesive used was the crosslinked Silicone adhesive gel from TA-B. TA-B was applied on an adhesive film identical to TB-A1. |
| TB-D0 | The gel-adhesive used was the crosslinked Silicone adhesive gel from TA-D. TA-D was applied directly on the leather. |
| TB-D1 | The gel-adhesive used was the crosslinked Silicone adhesive gel from TA-D. TA-D was applied on an adhesive film identical to TB-A1. |
| TB-E0 | The gel-adhesive used was the crosslinked Silicone adhesive gel from TA-E. TA-E was applied directly on the leather. |
| TB-E1 | The gel-adhesive used was the crosslinked Silicone adhesive gel from TA-E. TA-E was applied on an adhesive film identical to TB-A1. |

### Results:

| **Test Article** | **Adhesive bonding score** |
|---|---|
| TB-A0 | Type 3 |
| TB-A1 | Type 4 |
| TB-B0 | Type 2 |
| TB-B1 | Type 8 |
| TB-D0 | Type 3 |
| TB-D1 | Type 4 |
| TB-E0 | Type 0 |
| TB-E1 | Type 1 |

### Test result discussion:

Comparing TB-A0 with TB-A1, it is seen that the TA-A gel with lower crosslinking degree (and more unreacted polymer) only has limited synergistic effect (the adhesive bonding score increase from Type 3 to Type 4). Comparing TB-B0 with TB-B1, it is seen that the TA-B gel with medium crosslinking degree (and less unreacted polymer) has a high synergistic effect (the adhesive bonding score increase from Type 2 to Type 8). The reason for this is believed to be two-fold. Firstly, TA-B comprise less plasticizer (see gel-percentage). Secondly, the intra-structural breakdown of the gel in TA-B during the stress seems to be lower when comparing it to TA-A - due to the increased degree of crosslinking.

The consequence of adding oil can be seen if TB-B(0 and 1) is compared to TB-D(0 and 1). In a single adhesive construction (TB-B0 and TB-D0), the adhesive bonding score actually increases from a Type 2 to a Type 3 adhesive if 25% oil is added. Opposite, in a dual adhesive construction (TB-B1 and TB-D1), the synergistic effect is almost non-existing with 25% oil (the adhesive bonding score increases from a Type 3 to a Type 4 (TB-D1 compared to TB-D0)), but without oil added, the adhesive bonding score increases from a Type 2 to a Type 8 (TB-B1 compared to TB-B0)). This is a surprising effect, that lead to an argumentation in the present invention, that adhesives design for single adhesive construction are not optimal for dual-adhesive constructions.

When comparing TB-B(0 and 1), which is medium crosslinked with TB-E(0 and 1) it is clear that the gel off cause also can be too crosslinked. Consequently, there is an optimum in crosslinking degree (and HMDSO absorption).

### Example 3.

### Test Method C:

The 90-degree peel force was measured according to ISO 29862 (annex B), but where the leather (701) from Test method B was used instead of steel.

Each test article had an adhesive surface area of 25x100 mm. Each test measurement resulted in a (X,Y) data set, where X was elongation/pull of the test article (mm) and Y the corresponding force (N). The data set was plotted in a graph (see figure 6), where PEEL-force max, PELL-force average and the Modulus was measured.
PEEL-force max was the PEEL-force (N) measured when the crosslinked silicone adhesive gel started to separate from the leather. Since the test article has a width of 25mm, the unit is N/25mm
PELL-force average was measured as the average PEEL-force peeling 50-100 mm (N/25mm).

Modulus was measured as the slope of the graph (ΔN/Δmm) prior to PEEL-force max and based on the increase in force from 3-6 N (if the test articles had a PEEL-force max above 7 N/25mm) or 2-4 N (if the test articles had a PEEL-force max below 7 N N/25mm but above 4N) or 0.5-1.5 (if the test articles had a PEEL-force max below 4N but above 2N). Tests articles with a PEEL-force max below 2 N/25mm, Modulus was not applicable.

### Sample description:

| **Test Article** | **Description of test-set-up** |
|---|---|
| TC-B0 | The gel-adhesive used was the crosslinked Silicone adhesive gel from TA-B. TA-B was applied directly on the leather. |
| TC-B1 | The gel-adhesive used was the crosslinked Silicone adhesive gel from TA-B. Prior to application of TA-B, the leather was coated with Dow Corning MG 2401 from example B (solid content 30%) and subsequently, the solvent had evaporated. TA-B was applied on the remaining adhesive film on the leather. |
| TC-D0 | The gel-adhesive used was the crosslinked Silicone adhesive gel from TA-D. TA-D was applied directly on the leather. |
| TC-D1 | The gel-adhesive used was the crosslinked Silicone adhesive gel from TA-D. TA-D was applied on an adhesive film identical to TC-B1. |
| TC-E0 | The gel-adhesive used was the crosslinked Silicone adhesive gel from TA-E. TA-E was applied directly on the leather. |
| TC-E1 | The gel-adhesive used was the crosslinked Silicone adhesive gel from TA-D. TA-E was applied on an adhesive film identical to TC-B1. |

### Results:

| Test Article | PEEL-force Max (N/25mm) | PEEL-force Average (N/25 mm) | Modulus (ΔN/Δmm/25 mm) |
|---|---|---|---|
| TC-B0 | 17 | 11 | 0,58 |
| TC-B1 | 24 | 10 | 0,57 |
| TC-D0 | 15 | 7 | 0,37 |
| TC-D1 | 16 | 7 | 0,34 |
| TC-E0 | 2 | 2 | 1,05 |
| TC-E1 | 3,5 | 3 | 0,69 |

### Test result discussion:

In the above test, the most preferred second adhesive are TA-B in dual adhesive construction where focus are on maximal bonding strength.

As in Test Method B, TC-E1 illustrates that a too crosslinked adhesive gel (see HMDSO absorption) is not useful in the present invention, as the PEEL-force Max is too low and Modulus is too high which indicate it is not sufficiently elastic.

In this test, the synergistic increase in adhesive bonding strength of a dual-adhesive construction, where the crosslinking is optimized and plasticizer content kept low (TC-B1) can been in the increase in PELL-force Max (TC-B1 compared to TC-B0).

If plasticizer is added, (TC-D0 and TC-D1), the synergistic increase in adhesive bonding strength reduce or even disappear.

As modulus is lower for TC-D0 and TC-D1 (compared to TC-B0 and TC-B1), it is seen, that adding plasticizer to a gel-adhesive will as predicted increase softness.

### Overall test result discussion and conclusions:

Cross-linked Silicone adhesive gels with too high crosslinking degree (TA-E, TB-E1 and TC-E1) will not exercise sufficiently adhesive bonding in a dual adhesive construction (low adhesive bonding type score in Test Method B and low PEEL-force Max in test Method C). Unless they are added plasticizer the modulus (which reduce the bonding strength in a dual adhesive construction), their flexibility will be too low (their modulus will be too high).

Crosslinked Silicone adhesive gels with too low crosslinking degree will have a too low integrity and too low modulus.

Cross-linked Silicone adhesive gels with too much plasticizer (low Gel-percentage) will not exercise a significant synergistic adhesive strength effect in a dual adhesive construction compared to cross-linked Silicone adhesive gels with lower amount of plasticizer (high GEL percentage). See result on TA-B, TA-D; TB-B0, TB-B1, TB-D0, TB-D1. Similar in regards to PEEL-force max (TA-B, TA-D; TC-B0, TC-B1, TC-D0, TC-D1).

Test article TA-B and TA-C is a cross-linked Silicone adhesive gel according to the present invention. Test article TA-E (too high degree of crosslinking) is outside the scope of the present invention, while test article TA-A, TA-D and TA-F are borderline adhesive constructions, which may be useful in some cases (TA-A and TA-F due to a low crosslinking degree or HMDSO-absorption, and TA-D due to a high content of plasticizer or low GEL-percentage).

The synergistic effect of TA-B is very clear in Test method B, where the cross-linked Silicone adhesive gel increase from a type 2 in a single adhesive construction (TB-B0) to a type 8 in a dual adhesive construction (TB-B1).

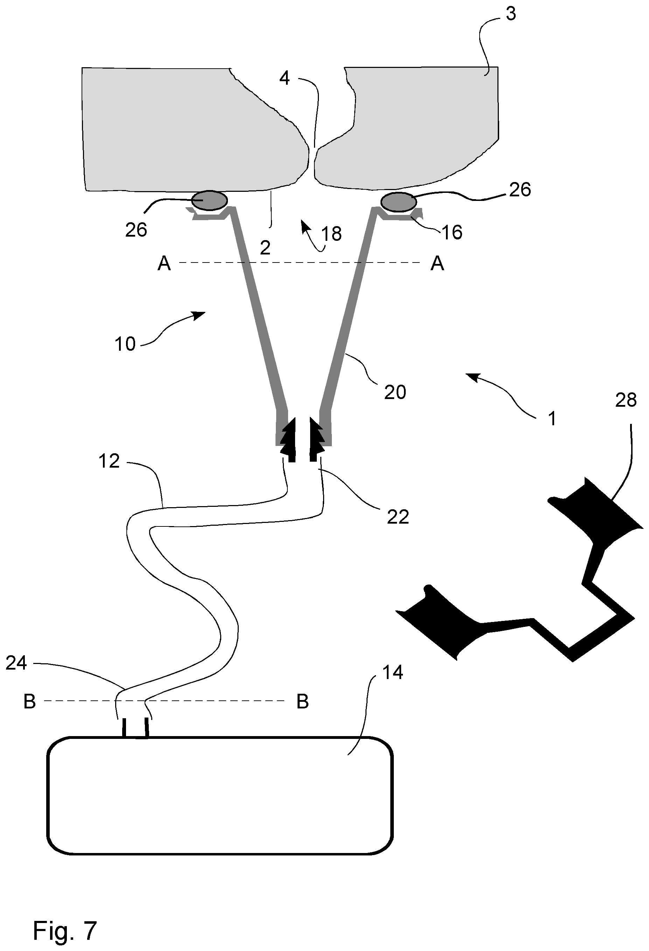

Figure 7 discloses a very schematic illustration showing the main components of the body waste collecting system in the form of a perianal faecal collecting system as an example of a medical system according to the current invention when attached to a user. It should be noted that in this current embodiment, the faecal collecting system is arranged to be used together with the adhesive types and systems as described above, however it should be clear to the person skilled in the art that the faecal collecting system could be used together with other forms of adhesive and/or mechanical fastening systems and/or combinations of adhesive and mechanical fastening systems.

The system 1 is arranged to be attached to the perianal skin 2 of a user 3 around the anal orifice 4 and collect faecal waste exiting the anus of the user. The system 1 in general comprises an attachment member 10, a conduit or tubular section 12 and a collection bag 14. The attachment member is arranged to establish a fluid tight connection between the system and the user. The conduit is arranged to establish fluid communication between the attachment member and the collection bag. The collection bag is arranged to collect the faecal output. This type of system is described in more detail in applicant's co-pending application PCT/EP2017/073345 which is included by reference in its entirety.

The attachment member 10 comprises an attachment flange 16 which is attached to the peri-anal skin of the user. The attachment member 10 of the current embodiment also comprises an opening 18 and an elongated portion 20. The conduit 12 has two ends, a first end 22 in fluid communication with the attachment member and a second end 24 in fluid communication with the collection bag.

In the current schematic embodiment, there are three separate elements, an attachment member 10, a conduit 12 and a collection bag 14. However, within the scope of the invention, these elements could be integrated together in different combinations. For example, an embodiment could be made which comprises a single integrated element. In another embodiment, the conduit and attachment member could be integrated into a single element, or the conduit and the collection bag could be integrated into a single element. Likewise, it should be mentioned that the conduit and the elongated portion of the attachment member fulfil similar functions. For the sake of the current specification and for the understanding of the claims, the attachment member should be understood as the part of the system which establishes a fluid tight communication with the anus or stoma of the user, the conduit should be understood as that part of the system which establish fluid communication between the attachment member and the collection bag, and the collection bag is the part of the system which collects the faecal or urinary waste from the user.

In the embodiment shown in figure 7, it can therefore be understood that the portion of the system which is above the line AA is considered the attachment member, the portion which lies under the line BB is considered the collection bag and the portion which lies between the lines AA and BB is considered the conduit. Using this understanding, in one embodiment the conduit should be at least 30 cm long and less than 8 cm in diameter. When compared to prior art solutions, this is typically longer and/or thinner than any systems which are attached externally to the perianal or peristomal skin.

In the current embodiment shown in figure 7, the attachment flange 16 is attached to the perianal skin via an adhesive 26 applied between the attachment flange and the perianal skin. Many suitable forms of adhesive have been discussed in the beginning of this application however other forms of adhesive could also be used. In another embodiment, instead of an adhesive, a mechanical system could be used with straps to press the attachment flange against the peri-anal skin. Adhesive and mechanical systems could also be combined if desired.

Figure 7 also shows an applicator 28 which in this embodiment is a stiff plastic injection moulded element which can be used to press the attachment flange 16 of the attachment member 10 against the perianal skin of the user. Once the attachment flange 16 is attached to the user 3, the applicator 28 can be removed and either discarded, or used again if necessary. Some examples of applicators are presented later on in this specification.

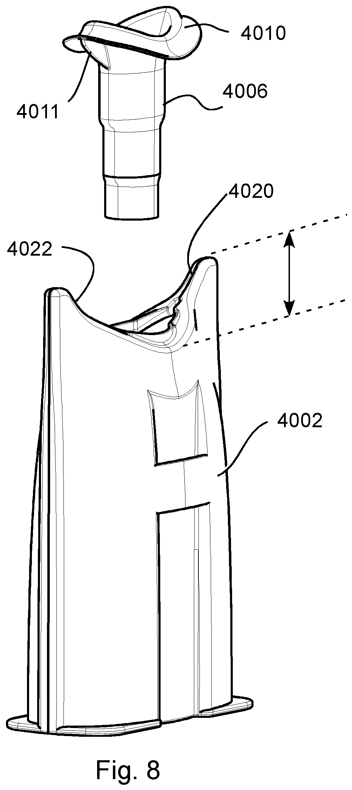

Figures 8 to 10 show different views of another embodiment 4000 of an applicator and attachment member. In this case, the figures mostly show an applicator 4002 and an attachment member 4006. It should be clear that a conduit would be attached to the base of the tubular section of the attachment member.

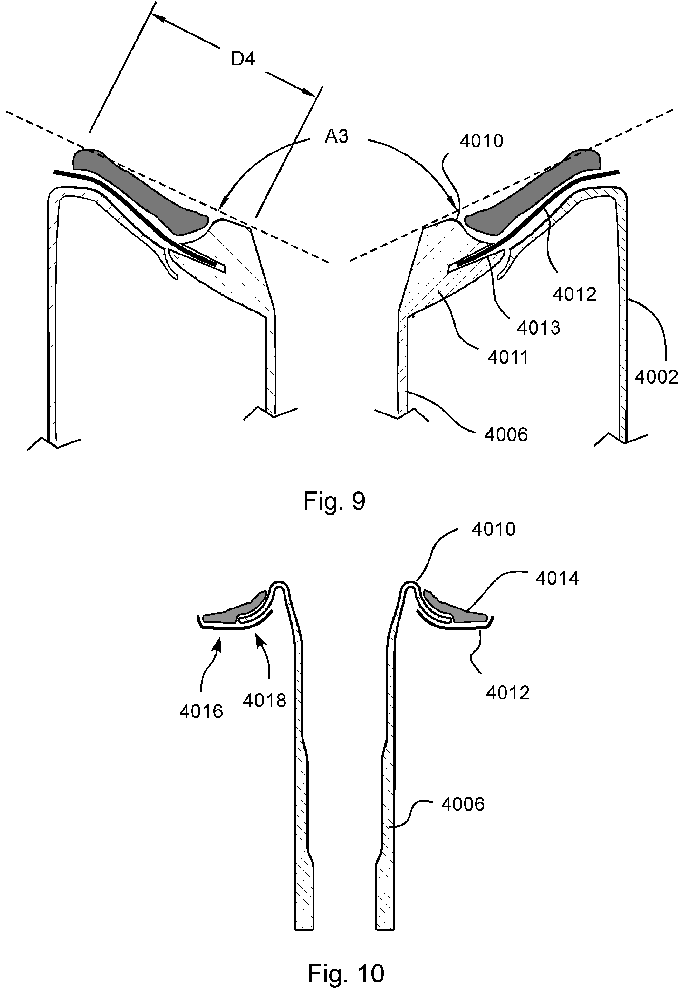

It should also be noted that in figure 8 only a portion of the attachment member 4006 is shown. In figures 9 and 10 the complete attachment member is shown. In this respect, it is noted that the attachment member in this embodiment, see figures 9 and 10, comprises a moulded silicon portion 4010, a non-woven flexible sheet portion 4012 and an adhesive 4014. The outer portion 4016 of the attachment flange is therefore very flexible due to the non-woven sheet while the inner portion 4018 of the flange is stiffer and supports the opening of the attachment flange from collapsing. In this embodiment, the two portions of the flange are provided as two separate elements joined together by an adhesive. However, in another embodiment (not shown), the inner and outer portions could be manufactured via a single injection moulding operation where the outer portion is thinner than the inner portion. This will have a similar effect. In another embodiment (not shown) instead of a non-woven outer portion, a foil could be used which is inserted into the injection mould prior to moulding the remaining portion of the attachment flange, similar to In Mould Label (IML) procedures.

In addition to the two part assembly of the attachment flange, the attachment member of the current embodiment comprises a vertically arranged supporting flange 4011 arranged at both the dorsal and ventral portions of the attachment flange. The supporting flanges 4011 help to push the dorsal and ventral portions into effective contact between the attachment flange and the Perineum and the Crena Ani during application. A slit 4013 is made in the supporting flanges to allow the non woven sheet 4012 to properly engage the moulded portion of the attachment flange. A slit also ensures that bending forces on the conduit are not transmitted to the attachment flange via the supporting flanges. The flanges with the slit only act to push the flange into contact with the perianal skin, not pull it way. In other embodiments, the vertically arranged supporting flange 4011 is not present.

In this embodiment, the attachment flange has a width of around 22mm all the way around the opening of the attachment member. Furthermore, the skin contacting surfaces of the attachment flange at the dorsal and ventral portions of the attachment flange form an angle to each other of around 130 degrees in this embodiment, see A3 in figure 9. This angle allows the dorsal and ventral portions to better contact the Crena Ani and Perineum portions of the user more effectively.

In the applicator 4002 shown in figures 8-10 the dorsal portion 4020 and ventral portion 4022 of the applicator have been made relatively long (see D4 in figure 9) and have a relatively thin cross section. In this way, when the applicator is applied to the user, the pressure applying surface of the applicator can apply pressure to the Perineum and the Crena Ani.

Furthermore, the pressure applying surfaces of the applicator have been elevated dorsally and ventrally with respect to the central portion. As with the attachment member, the elevation difference between the dorsal and ventral portions and the central portion creates an angle between the dorsal and ventral portions. In this embodiment, the angle between dorsal and ventral portions in figure 8 is approximately 135 degrees.

It should be noted that in this embodiment, the applicator is completely symmetric with regards to the dorsal/ventral portions. However in another embodiment (not shown), it could be that the dorsal portion is higher than the ventral portion.

In the above embodiments, applicators have been shown which are plastic injection moulded parts which have a pressure applying surface which extends around the entire opening. Thereby the applicator can be used to apply pressure to the attachment flange all the way around the opening of the attachment member. However, it could also be imagined that a more simple applicator was provided in some embodiments. For example, a bent metal rod of 4mm in diameter could be bent into a suitable shape, having a proximal handle portion and two distal pressure applying portions which engage with the dorsal and ventral portions of the attachment flange, but which do not contact the side portions of the attachment flange or the side portions of the tubular section. The conduit could be arranged between the two distal pressure applying portions. With this applicator, the attachment flange could be pressed into contact with the Perineum and the Crena Ani. Once the attachment flange was in contact with the Perineum and the Crena Ani, the sides of the attachment flange could be manually folded out to engage the skin of the user.

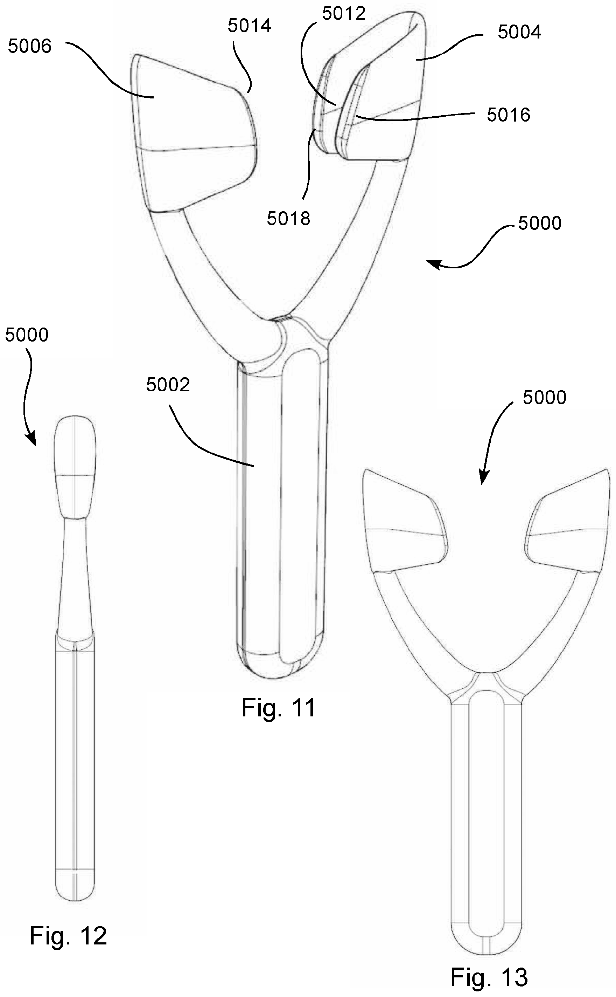

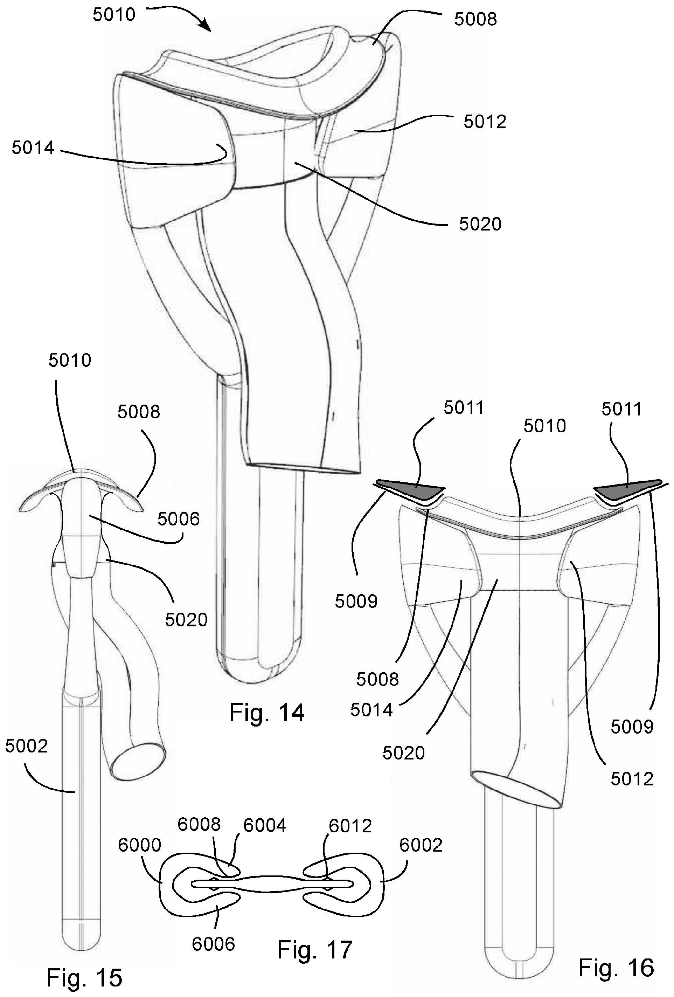

Figures 11-16 show some different views of another embodiment of an applicator for a feacal collection system as described above. In this example, the applicator 5000 is an injection moulded component having a handle portion 5002, a dorsal portion 5004 and a ventral portion 5006. As can be seen from figures 14-16, the applicator 5000 is arranged to support the attachment flange 5008 of an attachment member 5010 in a similar manner to the applicator of figures 8-10. As with figure 8, in figures 14-15, only a portion of the attachment member is shown. The complete attachment member will be similar to the one shown in figures 9 and 10 or as described above. In figure 16, the extra portions of the attachment flange are shown schematically. A flexible foil like element 5009 and an adhesive 5011 are shown. As was described above, these features could be provided in different ways. However, in one embodiment, the total stretchability of the attachment flange is high. Different example stretchabilities are discussed in the introduction of this specification. In the figures, a two part flange is shown with a base portion 5008 and a foil like portion 5009. However, as discussed above, this could also be provided as a single integrated part, possibly having different thicknesses to provide for different flexibilities.

In this embodiment of the applicator, instead of an oval opening which surrounds the entire attachment member, the opening is open at the sides so that only the dorsal and ventral portions of the attachment flange are supported. The side portions of the attachment flange are not supported. However, in practice it has been shown that once the attachment flange is well connected to the Perineum and Crena Ani of the user, the sides of the attachment flange will also be easy to attach to the user.

The applicator of the current embodiment, also comprises a flattening mechanism 5012,5014 which flattens the attachment member and/or the tubular section near the attachment member so that in the application of the attachment member 5010 to the user, the attachment member 5010 is flattened from its natural wider form to a more elongated or flat form which is easier to press in and attach to the user. In this embodiment, the flattening mechanism comprises a dorsal squeezing element 5012 and a ventral squeezing element 5014. Each of the two squeezing elements comprise two roughly parallel flanges 5016,5018 into which the tubular base portion 5020 of the attachment member can be squeezed. These two squeezing elements hold opposite sides of the tubular base portion 5020 of the attachment member and thereby hold it in a flattened position as shown in figures 14-16. This flattened position is especially illustrated in figure 15.

Figure 17 shows very schematically, the function of the squeezing elements of the flattening mechanism. The figure shows schematically, from above a ventral squeezing element 6000 and a dorsal squeezing element 6002. The two squeezing elements are both made from two opposing essentially parallel flanges 6004,6006. The two flanges are arranged to have a most narrow point 6008 towards the centre portion of the applicator. The tubular section 6010 is schematically shown in cross section through the longitudinal axis of the tubular section. As can be seen the tubular section has been flattened from its normal oval form to a flatten form to make it easier to apply to the user. The figure is shown very exaggerated to illustrate the concept better. On each side of the tubular section, there is a small ridge 6012 which forms a protrusion, thereby making the tubular section slightly thicker at these points. As can be seen in the figure, the ridges are held In place behind the narrowest point of the squeezing elements. In this way, the tubular section is held securely in place by the squeezing elements. However, when the ridges are pulled out of the squeezing elements, the remaining portion of the tubular section can be removed from the squeezing elements very easily. This ensures that the tubular section can be held both more securely in the applicator during application, but also be removed more consistently after application.

In this example the squeezing elements are arranged to hold the attachment member in place via friction. However, other forms of flattening mechanisms could also be imagined. For example instead of flattening the tubular section by squeezing opposite side portions of the tubular section, another form of mechanism could be provided which presses the central sides of the attachment member in towards each other to thereby make the attachment member narrower during the attachment process. One example could be a system which has two wires, one on each side of the attachment member. During application, the two wires could be moved towards each other to compress the attachment portion. The wires could also be arranged as a loop which is stretched during application such that the sides of the loop will be pressed together. Other mechanisms could also be provided.

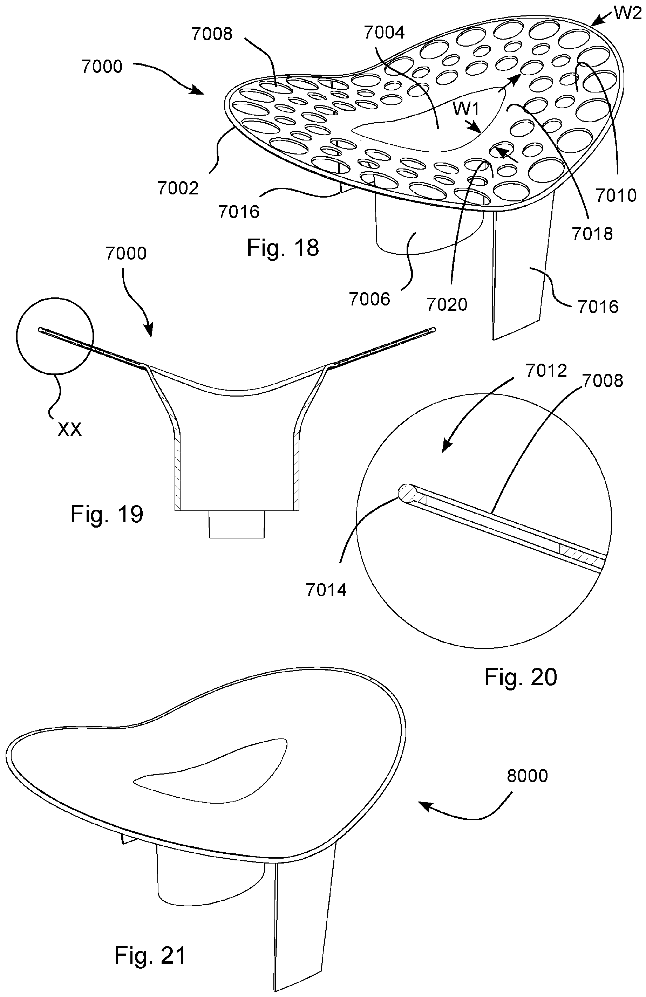

Figures 19 to 21 show different views of an attachment member 7000 of a body waste collecting system according to the second invention in the form of a faecal collecting system, or according to a fifth aspect of the first invention. The attachment member comprises an attachment flange 7002, an opening 7004 and a tubular portion 7006. The attachment flange is arranged to be suitable for attaching the attachment member to the perianal skin of a user. The attachment flange in this embodiment is provided with a number of through going holes 7008 in different sizes. The through going holes are arranged in patterns encircling the opening. Due to the through going holes, the stretchability and the flexibility of the attachment flange is increased significantly. In addition, due to the through going holes, the permeability of the attachment flange is increased significantly. This allows perspiration and moisture from the user to pass through the attachment flange portion without being trapped between the flange and the skin, or being absorbed by the adhesive. As was discussed previously, it has been found that perspiration can wander horizontally short distances, such that the perspiration which occurs under the solid portions of the attachment flange, can wander the relatively short distance to an opening to vent there.

In this embodiment, the through going holes pass all the way through the flange. A thin film (not shown) is therefore glued to the skin facing side 7010 of the attachment flange to "close" the holes. An adhesive can then be applied to the skin facing side of the thin film. The thin film is chosen from a material which is permeable, so that the perspiration can also vent through the thin film. Such film may be breathable Poly Urethane Films known from wound dressings (example Opsite 3000 from Smith+Nephew with a MVTR of 3000 g/m2/24h). Another example include breathable Silicone films such as Silpuran Film from Wacker. In one embodiment, the thickness of the film is less than 201 µm. In one embodiment, the thickness of the film is less than 101 µm. In one embodiment, the thickness of the film is less than 81 µm.

In one alternative example (not shown), instead of a thin film, a thin fabric or cloth like material is glued to the skin facing side of the attachment flange. If a cloth is used, a non woven may be used. Non-wovens typically have higher MVTR than films, but are also more permeable to bacteria and vira.

One could also use perforated (more occlusive) films. Suitable perforated films include micro-perforated films.

In the embodiment it can be seen that the attachment flange has a central portion 7018 and an outer portion 7020. The central portion encircles the opening and has a width of W1 while the outer portion encircles the central portion and has a width of W2. The central portion is free of holes which makes the permeability of the central portion much lower than the permeability of the outer portion. In this way gases having a undesirable odour are kept inside the attachment member and do not escape through the permeable outer portion of the attachment flange.

In one embodiment (not shown), instead of through going holes, the attachment flange is provided with recesses which do not pass completely through the flange. One could imagine an embodiment similar to the one shown in figure 18, but instead of through going holes, the holes shown in the figures would just be recesses or depressions in the attachment flange which do not go all the way through the flange. In this way, the moisture can still pass through the area of the recesses since the thickness of the material is reduced significantly. However, the adhesive is not directly accessible through the holes. In one embodiment the material thickness in the recesses is below 150 µm, 100 µm, or below 60 µm.

In another embodiment, a flange with through going holes is provided. Adhesive is applied on the skin facing side, and a thin breathable foil or cloth is applied on the surface of the attachment flange facing away from the skin. In this way, again, the adhesive is not accessible through the holes.

In the case where it is desired to provide a permeable attachment flange, it is also necessary to provide an adhesive which is also permeable. In one embodiment, the thickness of the adhesive on the flange is less than 1mm. By making the adhesive thin, the moisture can easily pass through the adhesive. In one embodiment, the thickness of the adhesive is less than 0.25 mm. In one embodiment, the thickness of the adhesive is less than 0.1 mm (100 µm). Suitable adhesives are in general all types of skin adhesive. Examples include polyacrylates and silicone skin adhesives.

In figure 20, the edge portion 7012 of the attachment flange 7002 is shown in more detail. Here one can see a through-going hole 7008. One can also see a slight circular bulge 7014 at the edge of the flange. This bulge is not necessary in all embodiments. In this current embodiment, the bulge can be used to provide a soft edge for the comfort of the user. Also, the edge can be used to provide a stop for the adhesive. The adhesive can then be applied inside the bulge.

Figure 18 also shows straps 7016 arranged on either side of the attachment flange. The straps are made from a flexible material, for example a cloth material or rubber like material. The straps are used to pull back the attachment flange during attachment to the user. The helper or the user pulls the straps backwards, thereby causing the sides of the attachment flange to lay flat against the sides of the tubular portion. The attachment member can then be more easily inserted against the perineum and Crena Ani. Once in place, the sides of the flange can be released and pushed outwards to attach to the side of the perianal skin. The straps also support the removal of the product after use.

It may be desirable to achieve strong adhesion to the perianal or peristomal skin. One way to achieve this would be to design an adhesive with a large surface contact to the skin. To achieve such large surface contact, a liquid smearable adhesive may be applied to the skin either as the sole adhesive or in combination with an adhesive on the attachment flange. In this way, the adhesive will fill the crevices in the skin and thereby achieve a very strong hold to the skin.

Figure 21 shows another embodiment 8000 of an attachment member which is very similar to the attachment member of figures 18-20. In this case, the attachment flange is a solid flange without any through going holes. However, the material has been chosen to be permeable and the thickness is reduced so much that the entire flange has a sufficient permeability as discussed above. In one embodiment, the average MVTR of the attachment flange is greater than 300 g/m2/24h.

It is to be noted that the figures and the above description have shown the example embodiments in a simple and schematic manner. Many of the specific mechanical details have not been shown since the person skilled in the art should be familiar with these details and they would just unnecessarily complicate this description. For example, the specific materials used and the specific fabrication techniques have not been described in detail since it is maintained that the person skilled in the art would be able to find suitable materials and suitable processes to manufacture the products according to the current invention.

Some explicit examples of medical systems, methods for producing a medical system, and methods for attaching a medical system according to the second invention are provided below.
Ex. 1. Body waste collecting system arranged to collect body waste from a user, said system comprising:
   a. an attachment member arranged to be attached to the user, said attachment member comprising an opening which is arranged to be in fluid communication with the anus or a stoma of the user and an attachment flange which is arranged to encircle said opening and which is arranged to be attached to the perianal or peristomal skin surrounding the anus or stoma of the user respectively such that a fluid tight seal is established between the attachment flange and the perianal or peristomal skin of the user entirely around the anus or the stoma respectively, said attachment flange having an outer width which is larger than the width of the conduit near the first end of the conduit, said attachment flange being provided with a skin friendly adhesive on a skin contacting surface of said attachment flange,
   b. a collection bag for collecting the body waste output, and
   c. a hollow conduit having a first end and a second end, said first end connected to said attachment member and being in fluid communication with the opening of said attachment member and said second end being in fluid communication with said collection bag,
   **characterized**
   d. in that the attachment flange has an average MVTR of at least 300 g/m2/24h.
Ex. 2. A body waste collecting system according to example 1, **characterized** in that the attachment flange comprises a number of recesses or through going holes arranged perpendicular to the plane of the attachment flange and arranged to encircle the opening of the attachment member.
Ex. 3. A body waste collecting system according to example 2, **characterized** in that the total surface area of the recesses and/or through going holes is greater than 10% of the total surface area of the attachment flange.
Ex. 4. A body waste collecting system according to examples 2 or 3, **characterized** in that the maximum distance between adjacent holes or recesses is less than 10mm.
Ex. 5. A body waste collecting system according to any one of examples 1 to 4, **characterized** in that the attachment flange comprises a central portion encircling the opening of the attachment member and an outer portion encircling the central portion, said central portion having a lower MVTR than said outer portion.
Ex. 6. A boy waste collecting system according to example 5, **characterized** in that the central portion has an MVTR of less than 750 g/m2/24h.
Ex. 7. A body waste collecting system according to example 5 or 6, **characterized** in that the outer portion has an MVTR of greater than 750 g/m2/24h.
Ex. 8. A body waste collecting system according to any one of examples 5-7, **characterized** in that said central portion is free of through going holes and/or recesses and the outer portion comprises recesses or through going holes.
Ex. 9. A body waste collecting system according to any one of example 5 to 8, **characterized** in that the width of the central portion is greater than 1mm and smaller than 12mm.
Ex. 10. A body waste collecting system according to any one of examples 1 to 9, **characterized i**n that the body waste collecting system is a faecal collecting system and the attachment member is arranged to be connected to the perianal skin around the anus of a user.
Ex. 11. A body waste collecting system according to example 10, **characterized** in that a dorsal portion and/or a ventral portion of the attachment flange is/are higher than a central portion of the attachment flange.
Ex. 12. A body waste collecting system according to any one of examples 10 or 11, **characterized** in that a dorsal portion and/or a ventral portion of the attachment flange extend dorsally at least 12 mm, at least 15mm, at least 18mm or at least 20mm from the dorsal/ventral portion of the opening respectively.
Ex. 13. A body waste collecting system according to any one of examples 1 to 12, **characterized** in that the m width of the attachment flange is greater than 8mm.
Ex. 14. A body waste collecting system according to any one of examples 1 to 13, **characterized** in that the body waste collecting system comprises a first adhesive attached to the user and a second adhesive attached to the attachment flange prior to attachment of the attachment member to the user.

## Claims

1. A medical system (100) comprising:
a. a solution comprising a first silicone adhesive (301) dissolved in a solvent suitable for application to the skin; said first silicone adhesive solution is adapted to leave an adhesive film (310) on the skin after application,
b. a medical device (200) having a surface comprising a second silicone adhesive (210) comprising a cross-linked silicone gel; and
**characterized in that** the second silicone adhesive (210) has a HexaMethylDiSilOxane absorption (HMDSO) of 1100 to 2500%.

2. The medical system according to claim 1, wherein the second silicone adhesive (210) has a Gel-percentage of 60 to 100.

3. The medical system according to claim 1 or 2, wherein the second silicone adhesive (210) has a Modulus of 0.35 to 0.65 N/mm.

4. The medical system according to any one of claims 1 to 3, wherein the second silicone adhesive (210) is attached in an average thickness of from 0.5 to 5.0 mm on a surface of the medical device.

5. The medical system according to any one of claims 1 to 4, wherein the first silicone adhesive solution is intended to leave a film on the skin, after the solvent has evaporated, said first adhesive film (310) having an average thickness of at most 0.5 mm.

6. The medical system according to any one of claims 1 to 5, wherein the concentration of the first silicone adhesive in the solution is from 45 to 65 w/w percent and a colourant is present in a concentration of 0.8 to 2.0 percent in the solution.

7. The medical system (100) according to any one of claims 1 to 6, wherein the medical device (200) comprises:
a. a non-replaceable part (220); and
b. optionally, a replaceable part (225), said replaceable part to be replaced more frequently than the non-replaceable part;
wherein the non-replaceable part comprises the second silicone adhesive (210).

8. A method for producing a medical system (100) according to any one of claims 1 to 7, the method comprising the steps of:
a. providing a solution comprising a first silicone adhesive (301) dissolved in a solvent suitable for application to the skin; said first silicone adhesive solution is adapted to leave an adhesive film (310) on the skin after application;
b. providing a medical device (200) having a surface comprising a second silicone adhesive (210) comprising a cross-linked silicone gel having a HexaMethylDiSilOxane absorption (HMDSO) of 1100 to 2500%, and
c. packing at least the medical device (200), and preferably the medical system (100).

9. A method for attaching a medical system (100) according any one of claims 1 to 7 to a skin surface (900), the method comprising the steps of:
a. applying a solution comprising a first silicone adhesive (301) dissolved in a solvent suitable for application to the skin on the skin surface (900);
b. evaporating a sufficient amount of the solvent to leave an adhesive film (310) on the skin; and
c. applying a second silicone adhesive (210) comprising a cross-linked silicone gel having a HexaMethylDiSilOxane absorption (HMDSO) of 1100 to 2500%, comprising a medical device (200), to at least a portion of the adhesive film (310) on the skin.

10. A dual adhesive system for attaching a medical device (200) to the skin (900), said dual adhesive system comprising:
a. a solution comprising a first silicone adhesive (301) being dissolved in a solvent for application on the skin; said first adhesive after application on the skin being adapted to leave an adhesive film (310) on the skin.
b. a second silicone adhesive (210) comprising a cross-linked silicone gel; and adapted to be attached to a medical device (200); and
**characterized in that** the second silicone adhesive (210) has a HMDSO-absorption of 1100 to 2500%.
